(19) **Europäisches Patentamt / European Patent Office / Office européen des brevets**

(11) **EP 4 306 694 B1**

(12) **EUROPEAN PATENT SPECIFICATION**

(45) Date of publication and mention
of the grant of the patent:
**07.05.2025 Bulletin 2025/19**

(51) International Patent Classification (IPC):
***D01H 13/32*** *(2006.01)*   ***G01N 21/86*** *(2006.01)*
***G01N 33/36*** *(2006.01)*   ***B65H 63/06*** *(2006.01)*
***G01N 21/89*** *(2006.01)*   ***D01G 31/00*** *(2006.01)*

(21) Application number: **22184635.5**

(22) Date of filing: **13.07.2022**

(52) Cooperative Patent Classification (CPC):
**D01H 13/32; B65H 63/06; D01G 31/006;
G01N 21/251; G01N 21/8915; G01N 33/365;
G01N 33/367; B65H 2701/31; G01J 3/50**

(54) **CONTROLLING OR ASSESSING THE MANUFACTURING OF YARN USING COLOR PARAMETERS AND YARN MANUFACTURING APPARATUS FOR PERFORMING THIS CONTROLL OR ASSESSING**

STEUERN ODER BEWERTEN DER GARNHERSTELLUNG UNTER VERWENDUNG VON FARBPARAMETERN UND SPINNVORRICHTUNG ZUR DURCHFÜHRUNG DIESES STEUERNS ODER BEWERTENS

CONTRÔLE OU ÉVALUATION DE LA FABRICATION DE FIL À L'AIDE DE PARAMÈTRES DE COULEUR ET MACHINE À FILER PERMETTANT CE CONTRÔLE OU CETTE ÉVALUATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**17.01.2024 Bulletin 2024/03**

(73) Proprietor: **Gebrüder Loepfe AG
8623 Wetzikon (CH)**

(72) Inventors:
• **Meier, Sandra
8340 Hadlikon (CH)**

• **Frick, Manuel Bruno
8610 Uster (CH)**
• **Hilzinger, Roger
8546 Islikon (CH)**

(74) Representative: **E. Blum & Co. AG
Franklinturm
Hofwiesenstrasse 349
8050 Zürich (CH)**

(56) References cited:
**EP-A1- 1 123 995      EP-A1- 1 371 978
EP-A1- 3 305 700      EP-A1- 3 499 222
JP-A- 2018 077 228    JP-A- 2021 001 427
US-A- 5 671 061       US-B1- 7 333 201**

**Description**

Technical Field

The invention relates to a method for controlling or assessing a yarn manufacturing process in a yarn manufacturing apparatus as well as to such an apparatus.

Background Art

Color has been known to be a relevant parameter of yarn. Hence, it may be measured in order to assess yarn quality.

In particular, sections of yarn not matching a desired color may be removed during the rewinding process in a yarn clearer. While such yarn clearing improves overall yarn quality, it leads to loss of time and material.

EP 3305700A1 describes a yarn winding system having yarn monitoring devices detecting yarn color defects.

EP 1123995A1 describes a textile fiber processing plant having cameras for detecting color changes.

Disclosure of the Invention

Hence, it is a general object of the invention to provide a method and apparatus of the type above that allow a better control the manufacturing process. Such better control may e.g. be obtained by an improved control or assessment of the process. In particular, the problem to be solved is to obtain a better understanding of the types of defects that may be present, and/or to judge if a given yarn fault is to be removed from the yarn or not.

This object is achieved by the method and apparatus of the independent claims.

Accordingly, the invention relates to a method for controlling or assessing a yarn manufacturing process in a yarn manufacturing apparatus having at least a first sensor head. The apparatus may e.g. be a spinning mill or a part thereof. Advantageously, the apparatus comprises several sensor heads, but some aspects of the present invention, e.g. some of the grouping, clustering, and/or correlation steps described below, can also be implemented with only a single sensor head as well.

The method comprises at least the following steps:

- Running an elongate textile body past the first sensor head: The textile body may e.g. be a yarn precursor or a yarn.
- Measuring, at a first plurality of times, by means of the first sensor head, at least one color parameter of the textile body: The color parameter is descriptive of the color of the yarn.
- Forming a time-series of values comprising the values of the color parameter. A "time-series" is to be understood as a series of values attributed to different times. These values at least include the values of the color parameter as well as, optionally, values of other parameters as mentioned below.
- Using the values of the color parameter for controlling or assessing the process.

The method is further characterized in that values that depend on the color parameter are clustered into color clusters.

In particular, the values can be used for at least one of the following measures:

a) They may be used for controlling a device of the yarn manufacturing apparatus;

b) They may be used to remove contaminants, unwanted components of the raw material, and/or sections with unwanted spatial arrangement of fibers (in this context, the "unwanted spatial arrangement of fibers" is understood to designate an undesired fiber geometry in the textile body, e.g. excessive hairiness or undesired conditions in compactness and/or linear density);

c) They may be used to alert an operator of a malfunction; and/or

d) They may be used for an automated or manual diagnosis of a malfunction.

The time-series may be "formed" on the fly, i.e. its values may not be stored permanently but e.g. be subject to immediate statistical pre-processing. Alternatively or in addition thereto, the measured values of the time-series may be stored permanently.

Advantageously, the method may comprise the steps of determining, from a time variation of the color parameter, at least one variation parameter and using this variation parameter for controlling or assessing the process: The variation parameter may e.g. be derived from the statistical variance of the values of the parameter, and/or it may be descriptive of color clusters formed by the values of the color parameter.

Advantageously, the method may further include the following steps:

- Determining, at a second plurality of times, in addition to the color parameter(s) of the textile body, at least one operating parameter of the apparatus: The operating parameter is e.g. a machine setting, a parameter of what goods are being processed by the apparatus, and/or an environmental parameter, such as air temperature or humidity. The second plurality of times may be the same as the first plurality of times, but it may also be a superset or subset thereof or it may be a different set of times, or the two sets may partially overlap.
- Processing the values of the operating parameter as part of the time-series of values. Such processing advantageously includes deriving information about the yarn manufacturing process from a combination of the values of the operating parameter and the color parameter.

In a particularly advantageous embodiment, the values of the operating parameter are permanently stored as part of the time-series of values, which allows for a later processing of the values.

Advantageously, for better comparing the values of the color parameter(s) and the values of the operating parameter(s), the first plurality of times (at which the color parameter is known) and the second plurality of times (at which the operating parameter is known) extend over at least a common time interval, i.e. the time ranges of the two pluralities of times overlap.

As mentioned, the operating parameter(s) may comprise at least one machine setting(s). For example, a given operating parameter may be one of the following:

- The operating parameter may be binary. In particular, it may be indicative of the on-off-state of a processing section of the apparatus. In this context, an on-off-state is a parameter describing if the given processing section is operating or not operating.
- The operating parameter by be categorial, i.e. descriptive of at least three categories. For example, it may be indicative of a raw material supplier or a color of yarn spun on a neighboring machine.
- The operating parameter may be quantitative and e.g. describe a machine setting during drafting (such as a distance between the drafting rolls) or the ambient humidity.

Using such a machine setting allows determining if the color parameter depends on the operating state of a processing section. For example, it may thus be found that operating a processing section might have a negative impact on another section of the apparatus.

The apparatus may comprise a plurality of individual processing sections, in which case the method can comprise the step of determining, for several of the processing sections, individual operating parameters. The parameters are individual in the sense that each parameter is descriptive of the operation at an individual processing section. Storing such individual operating parameters allows providing better insight into which processing sections have an influence on the values of the color parameter(s).

In a particularly important application, the operating parameter(s) and the color parameter(s) are correlated, i.e. it is assessed if the behavior, over time, of the color parameter(s) correlates with the operating parameter(s). This provides insight into how one or more operating parameters affect the color parameter(s).

For example, it may be found that the color parameter of a given elongate textile body may be correlated with the operating parameter of one or more processing sections that are not processing the given elongate textile body. This is particularly important for determining cross-contamination. For example, when processing a yarn of a given color and monitoring its color parameter, color faults may be correlated with the operation of sections of the apparatus processing a different yarn. In that case, it may be concluded that cross contamination is occurring between the section processing the given yarn and other sections processing other types of yarn.

According to the invention, the method comprises the step of grouping values that depend on the color parameter into color groups. This provides a better understanding of the types of defects that may be present, and/or it allows to judge if a given yarn fault is to be removed from the yarn or not.

The "values that depend on the color parameter" may be the individually measured values of the color parameter of values derived therefrom, e.g. values derived from a plurality of the measured values, for example by means of averaging.

The grouping is implemented by clustering, i.e. by an algorithm that does not require an a-priory knowledge of the groups used for grouping but may determine at least a subset of these groups (clusters) based on the values of the color parameter, e.g. by analyzing the distribution of the values of the color parameter. The clusters may be determined from starting parameters describing expected locations of the clusters, wherein at least some of the starting parameters are then refined to better match the measured values. Alternatively, the clusters may be determined based on the measured values alone, without using starting parameters for the cluster coordinates.

Hence, the method may comprise the step of clustering the values depending on the color parameter into clusters. Such clusters adapt to the current data, e.g. to the contaminants present at the site of the apparatus.

In this context, "clustering" into color clusters is the task of grouping the values of the color parameter in such a way that color values in the same group (called a cluster) are more similar to each other than to those in other groups (clusters). Similarity may e.g. be determined by a metric in the used color space, where the similarity increases as the distance derived from the metric decreases.

As mentioned, detecting cross contamination is an advantageous application of the present method. Therefore, for example, the method may comprise the following steps:

- Detecting at least one "potentially defective section" in a "first" elongate textile body: Such a potentially defective section may e.g. be a section where the color parameter deviates from the desired color parameter of the first textile body by a given threshold, or a number of sections identified from cluster analysis. Hence, advantageously, the values of the color parameter are used for detecting the potentially defective section(s).

- Comparing at least one "defect parameter" that is derived from the color parameter in the potentially defective section of the first elongate textile body against at least one "reference color" of a "second" elongate textile body processed in the apparatus. The "defect color" may be formed by values of the color parameter in the potentially defective section of the first elongate textile body. The reference color of the second elongate textile body may be the "desired color" of the second elongate textile body, or it may e.g. be the average color or median color of the second elongate textile body.

The second elongate textile body may be a textile body that fulfills at least one of the following conditions:

- The second textile body is processed in the apparatus concurrently with the first textile body. In this context, "concurrently" means that part of the second textile body are still being processed (e.g. manufactured or moved around or temporarily stored) in the apparatus when processing the potentially defective section. The detection of the potentially defective section may take place while processing it in the apparatus or in a later analysis of the data after processing the section.
- The second textile body has been processed in the apparatus prior to processing the first textile body. In this second case, advantageously, the time between processing the second and the first textile body is no more than a month, in particular no more than a week, in particular no more than a day. On the other hand, in some embodiments, the time between processing the second and the first textile body is at least an hour. In general, the time may depend e.g. on the cleaning interval of the apparatus, the processing of the air within the rooms when the apparatus operates, and/or other operating procedures.

The second elongate textile body advantageously has a "reference color" different from the desired color of first elongate textile body.

The reference color may be a parameter that has been entered through an interface into the apparatus, in particular by the user. Advantageously, though, the reference color is measured (i.e. derived from a measurement) by at least one of the sensor heads of the apparatus. This sensor head may be the "first sensor head" mentioned above (i.e. the same sensor head that is later used for measuring the first elongate body), or it may be another sensor head of the apparatus, i.e. a "second sensor head".

In order to compare the defect color against the reference color(s) of the second textile body (or bodies), the method may comprise the following steps:

- Determining the deviation between the defect color and the desired color of the first elongate textile body: This allows determining the "direction" of the defect in the used color space, such as "the yarn should be blue, but this defect is purple. It has a deviation towards more red and less blue".
- Comparing the deviation against the difference between

  - the reference color of the second textile body or bodies and
  - the reference color of the first textile body:
    A direct comparison of the defect color to the reference color of the second textile body would lead to less meaningful results than an indirect comparison by first calculating the deviation and then comparing the deviation to said difference in reference colors. In the example above, and assuming that the diameter is constant, blue fibers seem to have been replaced by red ones. If the reference color of the second yarn is red (or redder than the first yarn), the second yarn is a likely candidate for cross contamination.

Cross-contamination detection is particularly advantageous when it allows to identify one of several different "second textile bodies" as a potential source of cross contamination. Hence, advantageously, for detecting cross contamination, the defect color is compared against the reference colors of several different second elongate textile bodies processed in said apparatus. Based on this comparison, plausible sources of cross contamination are identified among the several different second elongate textile bodies. At least some of the several different second elongate textile bodies advantageously differ in their reference colors.

As mentioned, the invention also relates to a yarn manufacturing apparatus. This apparatus comprises a plurality of sensor heads and a control unit adapted and structured to carry out the method of the present invention.

Brief Description of the Drawings

The invention will be better understood and objects other than those set forth above will become apparent when consideration is given to the following detailed description thereof. Such description makes reference to the annexed drawings, wherein:

Fig. 1 is a schematic view of an apparatus for manufacturing yarn,
Fig. 2 shows an example of the normalized sensitivities of the color components measured by a sensor head,
Fig. 3 is schematic diagram of an embodiment of a sensor head,
Fig. 4 shows an example of the values of a color parameter as measured for textile body,

## Modes for Carrying Out the Invention

*Definitions*

*A color parameter* comprises at least two color components of a color space, advantageously at least three color components of a color space as measured by a sensor head. The color space is used to describe the color detected by the sensor head. In this context, the color space advantageously spans at least the visible spectrum, e.g. between wavelengths of 380 and 750 nm. It may, however, also extend into the ultraviolet, e.g. at least as low as 250 nm, and/or into the near infrared, e.g. at least as high as 1.8 $\mu$m.

For example, the components may describe the optical reflection or transmission of the textile body in at least two different spectral ranges. Advantageously, there may be at least three color components indicative of the optical reflection or transmission of the textile body in at least three different spectral ranges of the spectrum between, in particular with each of the at least three different colors falling at least in part into a spectral range of 250 nm and 1.8 $\mu$m, in particular at least in part into the visible spectral range of 380 and 750 nm.

The color space may also be the RGB color space or another color space indicative of the color in three or more different spectral ranges, or it may be the CMY or HSV color space, with the components being at least two of the coordinates in these color spaces.

*A textile body* may be a yarn precursor, in particular a stream of flocks in the blow room, or a sliver or roving as obtained by carding and drawing, or it may be a yarn as obtained by the spinning process. Advantageously, though, at least the textile body sampled at the claimed "first" sensor head is a yarn.

The *yarn manufacturing apparatus* comprises at least one of a blow room machinery, a carding machine, a drawing machine, a spinning machine, a winder, and a yarn clearer. "Blow room machinery" advantageously designates an opening machine or a cleaning machine, as they are e.g. found in the blow room.

Storing values *permanently* is advantageously to be understood as storing said values in non-volatile memory for at least a day.

*Overview*

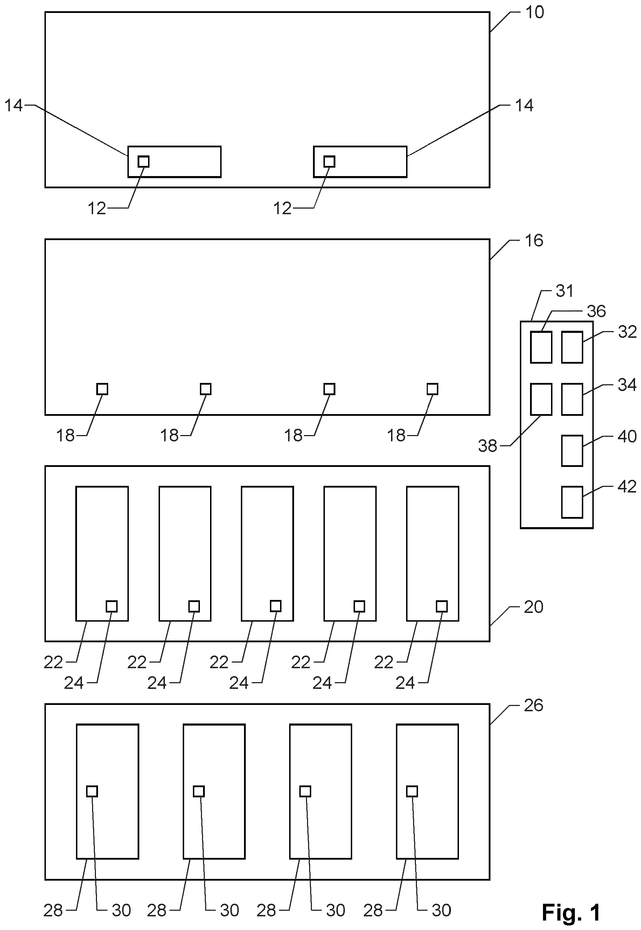

Fig. 1 schematically shows some elements of a cotton spinning mill as an example of a yarn manufacturing apparatus.

As known to the skilled person, such a spinning mill comprises a blow room 10, where bales of cotton are opened into flocks, which may then be precleaned as well as fine cleaned. In this process, one or more streams of flocks are generated.

The stream(s) of flocks may be run past sensor heads 12, e.g. in the fine cleaners 14 of the blow room 10. The function of these sensor heads 12 will be described below.

The mill further comprises a carding and drawing section 16, where the fibers in the flocks are separated, straightened, and formed into slivers and rovings. The slivers or rovings may again be run past sensor heads 18.

In a next step, the products from carding and rowing section 16 are fed to a spinning section 20, where they are spun into yarns in a plurality of spinning units 22. The yarns may again be run past sensor heads 24.

Finally, the yarns, e.g. wound on cops, are fed to a winding section 26 having a plurality of yarn clearers 28. In each yarn clearer 28, the yarn is run past a sensor head 30, e.g. while being wound from cops onto a bobbin.

The apparatus further comprises a control unit 31 controlling the operation of the individual components. In Fig. 1, it is depicted as a single element, but it may also be distributed and/or have sub-sections attributed to the different parts of the apparatus.

Typically, control unit 31 comprises at least one CPU 32, memory 34, input interfaces 36 for receiving sensor signals, e.g. from the various sensor heads as well as from other sensors of the apparatus, output interfaces 38 for controlling actuators of the apparatus, at least one input control 40 for receiving user input, and at least one display 42 for displaying information to the operator.

Control unit 31 may perform various functions using program code and parameters stored in memory 34. In particular, it is programmed to carry out the methods as described herein.

*Sensor heads*

As mentioned above, the apparatus comprises a plurality of sensor heads 12, 18, 24, 30. Each of these sensor heads is adapted to measure at least one color parameter $P_k$ of the textile body (which may be a yarn or yarn precursor as mentioned above) being run past it, with k = 1 ... K being an index of the sensor head and K being the number of sensor heads. The measurements carried out by the sensor heads take place online, i.e. they take place on a textile body while it is being processed by the apparatus.

The values of the color parameters are measured repetitively, at a plurality of different times $t_{ik}$, advantageously at regular time intervals.

Each color parameter comprises several color components $C_1 ... C_N$ of a color space, with N being at least 2, in particular at least 3. Each such color component $C_n$ may describe (i.e. may depend on) the interaction of the textile body with light of a given spectral range $W_n$, wherein the spectral ranges of the different color components differ from each other.

In one embodiment, the color space may be based on different spectral components in a spectral range as specified above.

Advantageously, each color component $C_n$ describes the optical reflectivity of the textile body in the given spectral range. However, in another embodiment, each color component $C_n$ may describe the optical transmission of the textile body at the given spectral range.

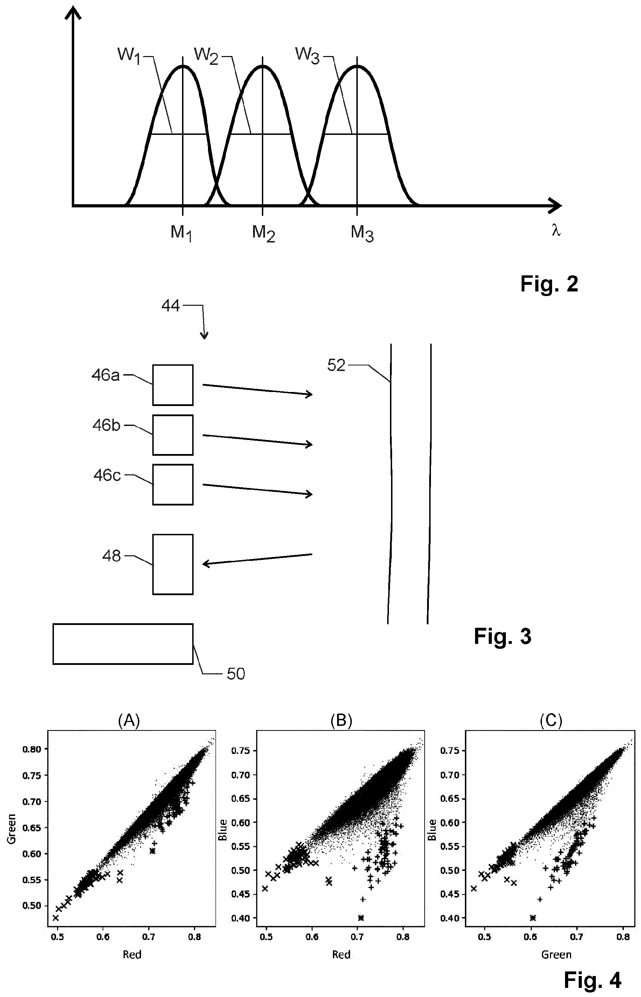

The spectral ranges are advantageously non-overlapping and/or span a substantial part of the visible spectrum. This is illustrated in Fig. 2, which shows the normalized sensitivities of the color components $C_n$ with n = 1 ... N (in this case for N = 3) as a function of the wavelength $\lambda$. For each color component, $W_n$ denotes the spectral range or width at half maximum and $M_n$ the wavelength of maximum sensitivity.

In order to be non-overlapping, there should advantageously be at least two, in particular at least three, color components whose spectral ranges $W_n$ have a mutual overlap by less than 0.25, with the mutual overlap $O_{ij}$ of two spectral ranges $W_i$ and $W_j$ being defined by

$$O_{ij} = 2 * W_{ij} / (W_i + W_j),$$

with $W_{ij}$ being the range where $W_i$ and $W_j$ overlap.

Advantageously, at least one spectral range, in particular at least two of them, should have a width $W_n$ smaller than 50 nm for good color selectivity.

In order to span a substantial part of the visible spectrum, there should advantageously be a least two, in particular at least three, color components whose maximum sensitivities $M_n$ are at least 50 nm away from each other.

Advantageously, at least one maximum sensitivity $M_n$ is in the violet, blue, or green spectral range, i.e. below 550 nm, and at least one maximum sensitivity $M_n$ is in the red spectral range, i.e. above 600 nm. In particular, there is advantageously one maximum sensitivity in the blue or violet spectral range below 500 nm, at least one maximum sensitivity in the green or yellow/orange spectral range between 500 nm and 600 nm, and at least one maximum sensitivity in the red spectral range above 600 nm.

Fig. 3 shows a schematic diagram of a possible embodiment of one sensor head 44. In the shown embodiment, it comprises three light sources 46a, 46b, 46c and a light detector 48. The light sources 46a, 46b, 46c emit light at different spectral ranges, corresponding e.g. to those in Fig. 2. Light sensor 48 is sensitive at all these spectral ranges. Control circuitry 50 is provided for operating the light sources 46a, 46b, 46c to e.g. emit light pulses sequentially and for detecting the response at light sensor 48 for each such light pulse.

The light from the light sources 46a, 46b, 46c falls on the textile body 52 to be inspected, interacts with the same, and, after this interaction, it is detected by light sensor 48. The interaction is advantageously a reflection, i.e. light sensor 48 detects light from the light sources 46a, 46b, 46c that has been reflected from textile body 52.

By attributing the measured light pulses to the individual light sources 46a, 46b, 46c, control circuitry 50 is able to measure the color components $C_n$ for the respective spectral ranges $W_n$.

In yet another embodiment (not shown), three separate light detectors sensitive only in the different spectral ranges $W_n$ may be used, e.g. in combination with a broadband light source.

An example of a suitable sensor head is e.g. described in EP3748343A1.

Different sensor heads 44 may be used for different types of textile bodies, i.e. bodies in different stages of the manufacturing process

However, since the color components as obtained by the various sensor heads may be compared to each other, as described below, in an advantageous embodiment, at least several of the sensor heads 44 are equipped to measure the same color components $C_n$, i.e. the color components $C_n$ determined by at least several of the sensor heads are the same components of a common color space. In particular, for a color space based on different spectral components, the spectral ranges $W_n$ and the maximum sensitivities $M_n$ of the several sensor heads are the same.

In particular, when the results from different types of yarn bodies in different stages of the yarn manufacturing apparatus are to be compared to each other (e.g. when the measurement on slivers are to be compared to those on yarns or flocks), the sensor heads used for measuring at different types of yarn bodies are advantageously adapted to measure the same color components $C_n$.

*Operation*

In operation, control unit 31 of the apparatus receives the values of the color parameters $P_k$ of the different sensor heads 44, with k being an index designating the respective sensor head as mentioned above. The number K of sensor heads typically being much larger than 1, in particular larger than 10.

Each color parameter $P_k$ comprises the color components $C_n$ determined by the respective sensor head k, or it may comprise different color components that are determined as a function of the color components $C_n$ actually measured by the sensor heads 44, e.g. color components may be translated into a different color space and/or values averaged over several individual measurements may be used.

Control unit 31 may store the values of the color parameters $P_k$ in memory 34, together with the times $t_{ik}$ to which they are attributed to, in particular at which they were measured. For example, assuming that N = 3, a table of the values of the components $C_{11}(t_{ik})$, $C_2(t_{ik})$, $C_3(t_{ik})$, and $t_{ik}$ may be stored for a given sensor head k in memory 34.

In addition, control unit 31 may store, in memory 34, individual operating parameters $O_m$ of the apparatus. These may include one or more of the following parameters:

A) Machine settings of the apparatus, i.e. values descriptive of the operating condition of an element of the apparatus. These may include one or more of the following parameters:

i) The on-off-state of a processing section of the apparatus. For example, such an on-off-state may describe if a given spinning unit 22 or a given yarn clearer 28 and its winding apparatus is presently running.

ii) The processing speed of a processing section of the apparatus, such as e.g. at least one of the following:

- the revolutions per time unit of the main cylinder of the cards (tambour),
- the production rate on the cards (material per time unit).

iii) The yarn tension in a processing section of the apparatus.

iv) Operating parameters of the drafting rolls.

v) Circular combing in the combing process.

vi) Drafting settings (distances and/or rotational speed) on combing machine.

vii) Drafting settings (distances and/or rotational speed) on drafting machines.

viii) Drafting settings (distances and/or rotational speed) on ring frame (main draft area).

ix) etc.

B) The type of good processed in a given element of the apparatus, in particular one or more of the following parameters:

i) The origin of the good being processed, e.g. the manufacturer of the cotton.

ii) The "desired color" and/or "reference color" (in the sense above) of the good being processed, e.g. the dyeing color of a yarn or yarn precursor.

iii) A parameter of the textile body, in particular of the yarn. This parameter may advantageously include the diameter of a yarn and/or the capacitance of a yarn that is being processed.

iv) etc.

C) One or more environmental parameter(s). This may e.g. include one of the following:

i) The temperature at one or more locations of the apparatus.

ii) The air humidity at one or more locations of the apparatus.

iii) etc.

The values of the operating parameter(s) $O_m$ with m = 1... M are determined at different times $t_{im}$ and may again be stored in memory 34.

The operating parameter(s) $O_m$ may be determined from control settings applied to the elements of the apparatus, from input data obtained by the operators of the apparatus, and/or from one or more sensors of the apparatus. For example, the diameter and/or capacitance of a yarn can be determined by suitable diameter and/or capacitance sensor heads of the apparatus.

The color parameters and operating parameters can then be processed by control unit 31 in various manner in order to derive information about the apparatus, to control its operation, to diagnose problems, and/or to display information.

Examples of such processing are provided in the following.

*Color grouping and clustering*

As mentioned, the values of one or more of the color parameters $P_k$, or values derived therefrom, are clustered.

This is illustrated with the example of Fig. 4, which shows values of the color parameter measured for a given textile body during a run. In this case, three color components $C_n$ were measured, one for a blue spectral range, one for a green spectral range, and one for a red spectral range. Each one of the dots, +-crosses, and x-crosses in Fig. 4 stands for the color components measured at one time $t_{ik}$, with i = 1 to I and I being the sum of the numbers of dots, +-crosses, and x-crosses.

As can be seen, the vast majority of the values (the ones that are shown as dots) are confined to a comparatively compact "main" group, but there are also two distinct "outlier" groups, one of which is denoted by the +-crosses and the other one by the x-crosses.

In the present case, the main group corresponds to color values close to the expected, desired color of the body while the outlier groups indicate specific, distinct defects in the body.

For example, the x-crosses, which are on the "darker" side of the main group, may be indicative of contamination with a dark contaminant while the +-crosses have a hue that is different from the one of the main group and may therefore be indicative of the contamination with a colored contaminant.

Hence, by grouping the values of the color parameter, additional insight into the nature of defects can be obtained, which allows to better control the manufacturing process. Furthermore, the decision-taking about what to do of the detected anomaly, especially if the textile body segment shall be cut out, can be dependent on the group or cluster membership of the anomaly.

The grouping is based on clustering, which is a dynamic process depending on the distribution of the values of the color parameter and may also rely on operating parameter(s) $O_m$, in particular on attributes of the textile body measured by other sensors. For example, the yarn diameter and/or capacitance may also be used for clustering, e.g. in order to group thickened sections of yarn having specific color characteristics and e.g. distinguishing them over (i.e. group them separate from) non-thickened sections having such color characteristics.

Suitable clustering algorithms are known to the skilled person, see e.g. https://en.wikipedia.org/wiki/Cluster_analysis, in particular one based on a centroid model, where each cluster is characterized by a central color. For example, k-means clustering may be used, see e.g. https://en.wikipedia.org/wiki/K-means_clustering, with k e.g. being set to 2 (if one primary contaminant is expected) and/or 3 (with two primary contaminants being expected).

Alternatively, algorithms using distribution-based clustering may be used, such as the expectation-maximum algorithm, where the colors are modeled with a fixed number of distributions, see e.g. https://en.wikipedia.org/wiki/Expectation%E2%80%93maximization_algorithm.

In yet another embodiment, density-based clustering may be used, where clusters are defined as areas of higher density than the remainder of the color data set, such as DBSCAN (density-based spatial clustering of applications with noise), see e.g. https://en.wikipedia.org/wiki/DBSCAN.

Advantageously, the grouping can also rely on prior knowledge about existing groups of contaminants and their typical color.

As mentioned, not each measurement of the color parameter $P_k$ is necessarily grouped or clustered. Rather, values may be derived from the individual measurements the color parameter $P_k$, e.g. by combining several measurements. For example, the mean of the color deviation over a fixed lengths of textile body or over variable-length abnormal sections may be calculated, and these mean values may than be grouped or clustered. Moreover, a prior decision criterion may be used to decide whether a segment is relevant enough to be clustered, dismissing values of other segments.

*Cross contamination detection*

As mentioned, a particularly advantageous technique relates to the detection of cross contamination, i.e. of a situation where particles of a material that is being processed or has been processed in the apparatus contaminate another material being processed.

Typically, a check for cross contamination is started when one or more potentially defective sections of a "first" elongate textile body is/are detected.

For example, potentially defective sections may be identified from the values of the color parameter, e.g.

using clustering as described in the previous section. In particular, an outlier group may be identified as defective sections sharing a same cause.

To check for cross contamination, the color of the potentially defective section, i.e. the "defect color", is compared (directly or indirectly) against at least one "reference color" of a second elongate textile body processed in said apparatus. The value of the reference color can e.g. be retrieved from the values of color parameters or process parameters as stored in memory 34.

For example, if a yarn (corresponding to the first elongate textile body) should be white, but the defect color indicates that it has a reddish tinge, memory 34 may be searched for indications that a reddish yarn (corresponding to the second elongate textile body) with a consistent hue has been processed or is being processed in the apparatus. If yes, cross contamination between this reddish yarn and the white yarn is a likely diagnosis, which then allows taking steps to avoid future cross contamination.

For example, if the used color space describes color values in three distinct spectral ranges as shown in Fig. 2, a defect color $V = (C_1, C_2, C_3)$ indicative of the potential defect may be derived from the color components in the potential defect or from a "typical color" of a cluster. This "typical color" may, for example, be the cluster's mean color, robust mean color, median color, and/or color of the centroid.

This defect color V can then be compared against reference colors attributed to various "second" textile bodies as stored in memory 34.

For example, memory 34 may comprise a list of the reference colors (e.g. average or median colors) $R_j$ with j = 1 to J of other textile bodies currently or recently processed in the apparatus.

In order for cross contamination to be most relevant, the reference colors $R_j$ should be different from the desired color of the first textile body. Cross contamination between two yarns of the same color are typically less relevant than cross contamination between two yarns of different color.

The comparison of the defect color V against the reference color(s) $R_j$ may comprise the following steps:

1) The deviation T of the defect color V from the desired color DC of the first elongate textile body is determined. The desired color DC may e.g. be the typical color of the main cluster as obtained by cluster analysis, or it may be derived from the average or median color of the first textile body, or it may be a value entered by the operator.

The deviation T can e.g. be calculated by vector subtraction of the color components, i.e.

$$T = V - DC.$$

For example, in RGB color space, if the yarn should be white (DC = (1.0, 1.0, 1.0)) and the defect color has a reddish tinge (e.g. V = (1.0, 0.7, 0.7)), T would be (0, -0.3, -0.3).

2) The deviation T is compared against the reference colors $R_j$. The mathematical details of this comparison depend on the used color space and the desired error tolerance of the process.

For example, the comparison may comprise the step of determining, for each reference color $R_j$, the step of calculating a "contaminant color correspondence value" $v_j$ and a "contaminant ratio" $w_j$ given by

$$v_j = T \cdot (R_j - DC) / (\|T\| \cdot \|R_j - DC\|),$$

$$w_j = T \cdot (R_j - DC) / \|R_j - DC\|^2,$$

with the first dot $\cdot$ denoting the scalar product of two vectors and with the vertical lines $\|...\|$ denoting an appropriate norm, e.g. the Euclidean norm, that is the length of a vector.

The contaminant color correspondence value $v_j$ is typically largest (close to 1) for the second textile body j (or bodies) that is/are likely to have given rise to cross contamination. It represents how well the hue of the color deviation matches a contamination by the textile body j. A contamination resulting in a mixture of fibers of the first elongate textile body and of fibers of the textile body j, the resulting color can be any color mixture in between the colors of the two textile bodies. $w_j$ represents the ratio of contaminant fibers, if the color results of such a mixture. Therefore, for a contamination to be plausible, $w_j$ must be between 0 and 1 after accounting for measurement uncertainty. In the example above, where T = (0, -0.3, -0.3), and memory 34 stores reference colors of a red textile body $R_1 = (1, 0, 0)$ and a green textile body $R_2 = (0, 1, 0)$), the correspondence value $v_1$ for the red body would be 1 while the correspondence value $v_2$ for the green body would be smaller than 1. Furthermore, the contaminant ratio for the red body is 0.3, which is a possible mixing ratio i.e. the red textile body is a likely candidate for cross contamination.

Hence, using this technique, potential sources of cross contamination can be identified.

Once one or more candidates have been identified, operating personnel of the apparatus may be able to determine when and how cross contamination occurred, and steps can be undertaken to reduce such cross contamination in the future, e.g. by identifying insufficient cleaning procedures of equipment or by spatially or temporarily separating problematic types of textile material.

In a particularly advantageous embodiment, cross-contamination detection is carried out for the yarns in the yarn clearers 28 during rewinding of the yarn. The rewinders and yarn clearers are often placed close to each other and cross-contamination, in particular between neighboring yarn clearers, has been found to be likely.

In another particularly advantageous embodiment, cross-contamination detection is carried out for the spinning units 22 based on measurements in the yarn clearers 28. Cross-contamination at that point leads to defects that are being spun into the yarn and that are hard to remove from the yarn without removing the contaminated sections.

Hence, advantageously, the first and second elongate textile bodies are being processed in the yarn clearers 28 of the apparatus

In order to assess the likeliness of cross-contamination, it is also advantageous to store the reference colors of the second elongate textile body or bodies together with the physical location where they have been processed in the apparatus. This allows assessing the spatial distance to the first textile body. A small distance is indicative of a higher risk of cross contamination.

Hence, advantageously, the method comprises the step of storing the reference color(s) of the second elongate textile body or bodies together with a location parameter indicative of a physical location where the second elongate textile body or bodies has or have been processed in the apparatus. This location parameter may e.g. be a unique id of a processing station in the apparatus.

*Other applications of grouping and clustering*

Grouping, and in particular, clustering, may not only provide a better understanding of cross contamination processes, but it may also be applied to other purposes.

For example, it can be used in yarn clearers for defining group-dependent processing rules. For example, it may be desired to provide stricter clearing rules, counting rules, or other processing rules for defects arising from foreign plastic materials than for defects arising from foreign organic materials. Foreign organic materials typically fall into a group that is in the brownish-yellow region of the color space while plastic materials can occur anywhere, which makes it possible to distinguish the two.

Hence, in more general terms, the invention may comprise the steps of

- grouping (in particular clustering) yarn defects as a function of the color and/or operating parameters, in particular parameters indicative of the electrical yarn capacity and/or the yarn diameter, into a different groups in a yarn clearer and
- applying different processing rules, in particular different yarn clearing criteria, for the different groups.

*Correlating parameters*

In yet another important technique, parameters may be correlated in order to detect the origin of problems.

In particular, the operating parameter(s) and the color parameter(s) may be correlated by control unit 31, i.e. it is assessed if the color parameter(s) correlates with the other operating parameter(s). This provides insight into how one or more operating parameters affect the color parameter(s).

Correlation techniques are known to the skilled person, see e.g. https://en.wikipedia.org/wiki/Correlation.

Hence, advantageously, the current method comprises the step of determining the correlation between

- a value $VO_m$ depending on at least one operating parameter and
- a value $VP_k$ depending on the color parameter.

For example, let us assume that the values of the (first) color parameter $P_k$ has been recorded at times $t_{ik}$ and stored in memory 34.

For example, assuming that N = 3, a table of the values of the components $C_1(t_{ik})$, $C_2(t_{ik})$, $C_3(t_{ik})$, and $t_{ik}$ has been stored. Alternatively to explicitly storing the times $t_{ik}$, other parameters may be stored, such as

- an index of the measurement, or
- a position value indicating as to where along the textile body the components have been recorded. This may e.g. include a cops or bobbin index as well as a position along the yarn that is wound onto the cops or bobbin.

In addition, one or more operating parameter(s) $O_m(t)$ as described above with m = 1... M has/have been determined at different times $t_{im}$ and their values are again stored in memory 34.

In that case, the correlation between values $VP_k$ derived from color parameter $P_k$ and values $VO_m$ derived from the operating parameter(s) $O_m$ may be calculated.

Advantageously only one of the time series $VO_m$ and $VP_k$ is stored and the correlation is calculated live while processing the second time serie.

For example, the value $VP_k$ may correspond to one of the color components $C_n$. Advantageously, though, it depends on more than one of the color components $C_n$ in order to use the information embedded in several components. For example, it may be calculated from the absolute values of the deviations of the color component $P_k$ from the desired color DC as described above, i.e.

$$VP_k = | P_k - DC|.$$

The $VP_k(t1)$ for a given time t1 may also be calculated from several measured color parameters $P_k$ at different times around t1. In addition or alternatively thereto, the value $VO_m(t2)$ for a given time t2 may also be calculated from several operating parameters $O_m$ at different times around t2. These values may e.g. be determined using rolling averages, interpolation and/or other data combination techniques.

In yet another embodiment, the values $VP_k(t1)$ may e.g. be the number of color faults per yarn length or the number of faults classified in a given cluster for a given section of textile body.

The times t1 and t2 for which the values $VP_k(t1)$ and $VO_m(t2)$ are to be correlated depend on the stage of the production chain the parameters are associated with. For example, if an operating parameter $O_m$ of a spinning section 20 affects the color parameter $P_k$ of a yarn as measured in a yarn clearer 28, t2 needs to be at a suitable time before t1. Also, it may be necessary to take into account the different processing speeds of the various processing stages of the spinning mill in order to correlate parameters applying to the same section of a textile body.

Hence, generally, the correlation between the value $VP_k(t1)$ attributed to a first time t1 and the value $VO_m(t2)$ attributed to a second time t2 has to be determined, with t1 = $f_{km}(t2)$. The function $f_{km}$ describes how the times $t_{im}$ of the known values of the operating parameter(s) $O_m(t)$ are related to the times $t_{ik}$ of the measurements of the color parameters.

Typically, the function $f_{km}$ is as follows:

$$f_{km}(t2) = \Delta t + k \cdot t2,$$

with $\Delta t$ describing the time delay between the stage the operating parameter is assigned to (e.g. spinning) and the stage where the color parameter is measured (e.g. rewinding in the yarn clearer). k is the ratio of the different yarn processing speeds of at the first and second stage. $\Delta t$ and k are calculated such that the color parameter $P_k$ at a given location on the yarn is correlated with the operating parameter $O_m$ when that same location was processed in the first unit.

The correlation between $VP_k(f_{km}(t2))$ and $VO_m(t2)$ describes if the operating parameter $O_m$ has an influence on the color parameter $P_k$.

Hence, in more general terms, the present invention may comprise the step of determining the correlation of at least one value derived from a color parameter at a given first time t1 with at least one value derived from an operating parameter recorded at a second time t2, with the second time being before the first time.

*Cross-correlating parameters*

The correlation technique described in the previous section assumes that the time delay between the measuring times of the color parameters and the operating parameters is known, i.e. that the function $f_{km}$ is known. Sometimes, however, it may not be known which previous process step in the production chain has an impact on a measured color parameter $P_k$. For example, humidity and/or temperature may have affected any one of several previous manufacturing steps of a yarn for which a color parameter is determined.

In this case, cross-correlation techniques can be used to identify if the color parameter $P_k$ depends on an operating parameter $O_m$ at an unknown, previous time, see e.g. https://en.wikipedia.org/wiki/Cross-correlation. Cross-correlation provides additional information over mere correlation since it also allows determining a time delay between the similar traits.

In that case, a value $VP_k(t)$ derived from color parameter $P_k$ may be subjected to cross-correlation with a value $VO_m(t)$ derived from the operating parameter(s) $O_m$, e.g. by calculating the integral

$$\int VP_k(t) \cdot VO_m(t + \tau)\, dt$$

*Notes*

Hence, in the present system, a yarn is manufactured in a yarn manufacturing apparatus having a plurality of sensor heads 20, 18, 24, 30, 44. At least one variation parameter is determined from the values of the color parameter as a function of time. This variation parameter may e.g. be obtained from clustering or from cross correlation or from the deviation of the color values from a desired color as described above.

The variation parameter can then be used for assessing the process, i.e. for diagnosing possible causes for problems, and/or it can be used to control the process. The process can be controlled directly by control unit 31 after it has analyzed the variation parameter, or the control can be based in operator input after the operator has analyzed the variation parameter.

While there are shown and described presently preferred embodiments of the invention, it is to be distinctly understood that the invention is not limited thereto but may be otherwise variously embodied and practiced within the scope of the following claims.

**Claims**

1. A method for controlling or assessing a yarn manufacturing process in a yarn manufacturing apparatus having at least a first sensor head (12, 18, 24, 30; 44), wherein the method comprises the steps of

    running an elongate textile body (52) past the first sensor head (12, 18, 24, 30; 44),
    measuring at a first plurality of times, by means of the first sensor head (12, 18, 24, 30; 44), a color parameter ($P_k$) of the textile body (52), forming a time-series of values comprising values of the color parameter ($P_k$) attributed to different times,

using the values of the color parameter ($P_k$) for controlling or assessing the process,
**characterized in that** values ($VP_k$) that depend on the color parameter ($P_k$) are clustered into color clusters.

2. The method of claim 1 further comprising the steps of

    determining, from a time variation of the color parameter, at least one variation parameter and using this variation parameter for controlling or assessing the process.

3. The method of any of the preceding claims further comprising the steps of

    determining, at a second plurality of times, in addition to the color parameter ($P_k$) of the textile body (52) in said time-series, at least one operating parameter ($O_m$) of the apparatus and processing values of said operating parameter ($O_m$) in the time-series of values, and in particular wherein the values of the operating parameter ($O_m$) are permanently stored as part of the time-series of values.

4. The method of claim 3 wherein the first plurality of times and the second plurality of times extend over at least a common time interval.

5. The method of any of the claims 3 or 4 wherein the operating parameter ($O_m$) comprises at least a machine setting of the apparatus.

6. The method of claim 5 wherein said machine setting(s) comprise(s) at least one of

    an on-off-state of a processing section of the apparatus,
    a raw material supplier,
    a color of yarn spun on a neighboring machine, and
    a machine setting during drafting, and ambient humidity.

7. The method of any of the claims 3 to 6 wherein the apparatus comprises a plurality of processing sections, and wherein the method comprises determining, for several of the processing sections, individual operating parameters ($O_m$).

8. The method of claim 7 wherein the textile body (52) is a yarn of a given color and wherein the values ($VO_m$) depending on the operating parameters ($O_m$) of processing sections that are not processing the yarn of the given color (52) are correlated with the value ($VP_k$) depending on the color parameter ($P_k$) of yarn.

9. The method of any of any of the preceding claims comprising the step of cross-correlating a value $(VO_m)$ depending on the operating parameter $(O_m)$ and a value $(VP_k)$ depending on the color parameter $(P_k)$.

10. The method of any of the preceding claims comprising the steps of

    grouping, in particular clustering, yarn defects as a function of the color parameter $(P_k)$ and/or an operating parameter, in particular parameter indicative of the electrical capacity and/or diameter of the yarn, into a different groups in a yarn clearer, and
    applying different processing rules, in particular different yarn clearing criteria, for the different groups.

11. The method of any of the preceding claims comprising the steps of

    detecting, using the values of the color parameter, at least one potentially defective section in a first elongate textile body (52),
    for detecting cross contamination, comparing at least one defect color (V) derived from the color parameter $(P_k)$ in the potentially defective section against at least one reference color $(R_j)$ of a second elongate textile body (52) processed in said apparatus.

12. The method of claim 11 wherein the second elongate textile body (52) is processed, in said apparatus, concurrently with the first textile body (52).

13. The method of claim 11 wherein the second elongate textile body (52) has been processed, in said apparatus, prior to processing the first textile body (52), and in particular wherein

    a time between processing the second and the first textile body (52) is no more than a month, in particular no more than a week, in particular no more than a day and/or
    a time between processing the second and the first textile body (52) is at least an hour.

14. The method of any of the claims 11 to 13 wherein the second elongate textile body (52) has a reference color $(R_j)$ different from the first elongate textile body (52).

15. The method of any of the claims 11 to 14 wherein the reference color $(R_j)$ is measured by the apparatus by means of the first sensor head (12, 18, 24, 30; 44) or by means of a second sensor head (12, 18, 24, 30; 44) of the apparatus.

16. The method of any of the claims 11 to 15, wherein, for comparing the defect color (V) against the at least one reference color $(R_j)$, the method comprises the steps of

    determining a deviation (T) between the defect color (V) and a desired color (DC) of the first elongate textile body (52) and
    comparing the deviation (T) against a difference between the reference color $(R_j)$ and a reference color of the first textile body (52).

17. The method of any of the claims 11 to 16 for detecting cross contamination, the defect color (V) is compared against a difference between the reference color $(R_j)$ of several different second elongate textile bodies processed in said apparatus and a reference color of the first textile body.

18. The method of any of the claims 11 to 17 wherein the reference color $(R_j)$ is an average color or a median color of the second elongate textile body or bodies (52).

19. The method of any of the claims 11 to 18 wherein the first and second elongate textile bodies (52) are being processed in yarn clearers (28) of the apparatus.

20. The method of any of the claims 11 to 19 comprising the step of storing the reference color(s) $(R_j)$ of the second elongate textile body or bodies (52) together with a location parameter indicative of a physical location where the second elongate textile body or bodies (52) has or have been processed in the apparatus.

21. The method of any of the preceding claims wherein the color parameter $(P_k)$ comprises at least two color components $(C_n)$, in particular at least three color components $(C_n)$ of a color space.

22. The method of claim 21 wherein the components $(C_n)$ describe an optical reflection or transmission of the textile body (52) in at least two different spectral ranges $(W_n)$, in particular in at least three different spectral ranges $(W_n)$, and wherein the color components have maximum sensitivities $(M_n)$ at three different wavelength.

23. The method of claim 22 wherein

    the different spectral ranges $(W_n)$ have a mutual overlap of less than 0.25, and/or
    at least one spectral range $(W_n)$, in particular two of them, has or have a width smaller than 50 nm, and/or
    at least one maximum sensitivity $(M_n)$ is below

550 nm and at least one maximum sensitivity $(M_n)$ is above 600 nm, and in particular at least one maximum sensitivity $(M_n)$ is below 500 nm, at least one maximum sensitivity $(M_n)$ is between 500 nm and 600 nm, and at least one maximum sensitivity $(M_n)$ is above 600 nm.

24. The method of any of the preceding claims wherein the yarn manufacturing apparatus comprises a plurality of sensor heads (17, 18, 24, 30; 44).

25. The method of any of the claims 21 to 23 and of claim 24 wherein at least several of the sensor heads (12, 18, 24, 30; 44) measure the same color components $(C_n)$.

26. The method of claim 25 wherein sensor heads (12, 18, 24, 30; 44) used for measuring at different types of yarn bodies measure the same color components $(C_n)$.

27. The method of any of the preceding claims comprising the step of permanently storing the time-series of values.

28. A yarn manufacturing apparatus comprising

at least one first sensor head (12, 18, 24, 30; 44) and
a control unit (31) adapted and structured to carry out the method of any of the preceding claims.

**Patentansprüche**

1. Verfahren zum Steuern oder Beurteilen eines Garnherstellungsprozesses in einer Garnherstellungsvorrichtung mit mindestens einem ersten Sensorkopf (12, 18, 24, 30; 44), wobei das Verfahren die folgenden Schritte umfasst

Vorbeiführen eines langgestreckten Textilkörpers (52) am ersten Sensorkopf (12, 18, 24, 30; 44),
Messen eines Farbparameters $(P_k)$ des Textilkörpers (52) zu einer ersten Mehrzahl von Zeitpunkten mittels des ersten Sensorkopfes (12, 18, 24, 30; 44),
Bildung einer Zeitreihe von Werten, die verschiedenen Zeitpunkten zugeordnete Werte des Farbparameters $(P_k)$ umfassen,
Verwendung der Werte des Farbparameters $(P_k)$ zur Steuerung oder Beurteilung des Prozesses,
**dadurch gekennzeichnet, dass** Werte $(VP_k)$, die vom Farbparameter $(P_k)$ abhängen, zu Farb-

clustern zusammengefasst werden.

2. Verfahren nach Anspruch 1 umfassend weiter die folgenden Schritte

Bestimmung mindestens eines Variationsparameters aus einer zeitlichen Veränderung des Farbparameters und
Verwendung dieses Variationsparameters zum Steuern oder Beurteilen des Prozesses.

3. Verfahren nach einem der vorhergehenden Ansprüche umfassend ferner die Schritte

Bestimmen, zu einer zweiten Mehrzahl von Zeitpunkten, zusätzlich zu dem Farbparameter $(P_k)$ des Textilkörpers (52) in der Zeitreihe mindestens eines Betriebsparameters $(O_m)$ der Vorrichtung und
Verarbeiten der Werte des genannten Betriebsparameters $(O_m)$ in der Zeitreihe der Werte, und insbesondere wobei die Werte des Betriebsparameters $(O_m)$ als Teil der Zeitreihe von Werten dauerhaft gespeichert werden.

4. Verfahren nach Anspruch 3, wobei sich die erste Mehrzahl von Zeitpunkten und die zweite Mehrzahl von Zeitpunkten über mindestens ein gemeinsames Zeitintervall erstrecken.

5. Verfahren nach einem der Ansprüche 3 oder 4, wobei der Betriebsparameter $(O_m)$ mindestens eine Maschineneinstellung der Vorrichtung umfasst.

6. Verfahren nach Anspruch 5, wobei die Maschineneinstellung(en) mindestens eines der folgenden Elemente umfasst (umfassen)

einen Ein-Aus-Zustand eines Verarbeitungsteils der Vorrichtung,
einen Rohstofflieferanten,
eine Farbe des auf einer benachbarten Maschine gesponnenen Garns und
eine Maschineneinstellung während des Streckens, und Luftfeuchtigkeit.

7. Verfahren nach einem der Ansprüche 3 bis 6, wobei die Vorrichtung eine Mehrzahl von Verarbeitungsabschnitten umfasst, und wobei das Verfahren die Bestimmung einzelner Betriebsparameter $(O_m)$ für mehrere der Verarbeitungsabschnitte umfasst.

8. Verfahren nach Anspruch 7, wobei der Textilkörper (52) ein Garn einer bestimmten Farbe ist und wobei die von den Betriebsparametern $(O_m)$ der Bearbeitungsabschnitte, die das Garn der bestimmten Farbe (52) nicht bearbeiten, abhängigen Werte $(VO_m)$ mit dem vom Farbparameter $(P_k)$ des Garns abhängi-

gen Wert ($VP_k$) korreliert werden.

9. Verfahren nach einem der vorhergehenden Ansprüche umfassend den Schritt der Kreuzkorrelation eines Wertes ($VO_m$), der vom Betriebsparameter ($O_m$) abhängt, und eines Wertes ($VP_k$), der vom Farbparameter ($P_k$) abhängt.

10. Verfahren nach einem der vorhergehenden Ansprüche umfassend die Schritte

     Gruppieren, insbesondere Clustern, von Garnfehlern in Abhängigkeit des Farbparameters ($P_k$) und/oder eines Betriebsparameters, insbesondere eines Parameters, der die elektrische Kapazität und/oder den Durchmesser des Garns angibt, in verschiedene Gruppen in einem Garnreiniger, und
     Anwendung unterschiedlicher Verarbeitungsregeln, insbesondere unterschiedlicher Kriterien für die Garnreinigung, für die verschiedenen Gruppen.

11. Verfahren nach einem der vorhergehenden Ansprüche umfassend die Schritte

     Erkennen mindestens eines potenziell fehlerhaften Abschnitts in einem ersten langgestreckten Textilkörper (52) anhand der Werte des Farbparameters,
     zum Erkennen von Kreuzkontaminationen, Vergleichen mindestens einer Fehlerfarbe (V), die aus dem Farbparameter ($P_k$) in dem potenziell fehlerhaften Abschnitt abgeleitet wird, mit mindestens einer Referenzfarbe ($R_j$) eines zweiten länglichen Textilkörpers (52), der in der Vorrichtung verarbeitet wird.

12. Verfahren nach Anspruch 11, wobei der zweite längliche Textilkörper (52) in der Vorrichtung gleichzeitig mit dem ersten Textilkörper (52) bearbeitet wird.

13. Verfahren nach Anspruch 11, wobei der zweite längliche Textilkörper (52) in der Vorrichtung vor der Verarbeitung des ersten Textilkörpers (52) verarbeitet wurde, und insbesondere wobei

     eine Zeitspanne zwischen der Bearbeitung des zweiten und des ersten Textilkörpers (52) nicht mehr als einen Monat, insbesondere nicht mehr als eine Woche, insbesondere nicht mehr als einen Tag beträgt und/oder
     eine Zeitspanne zwischen der Bearbeitung des zweiten und des ersten Textilkörpers (52) mindestens eine Stunde beträgt.

14. Verfahren nach einem der Ansprüche 11 bis 13, wobei der zweite langgestreckte Textilkörper (52)

eine Referenzfarbe ($R_j$) aufweist, die sich von der des ersten langgestreckten Textilkörpers (52) unterscheidet.

15. Verfahren nach einem der Ansprüche 11 bis 14, wobei die Referenzfarbe ($R_j$) von der Vorrichtung mittels des ersten Sensorkopfes (12, 18, 24, 30; 44) oder mittels eines zweiten Sensorkopfes (12, 18, 24, 30; 44) der Vorrichtung gemessen wird.

16. Verfahren nach einem der Ansprüche 11 bis 15, wobei das Verfahren zum Vergleichen der Fehlerfarbe (V) mit der mindestens einen Referenzfarbe ($R_j$) die Schritte umfasst

     Bestimmen einer Abweichung (T) zwischen der Fehlerfarbe (V) und einer gewünschten Farbe (DC) des ersten länglichen Textilkörpers (52) und
     Vergleichen der Abweichung (T) mit einer Differenz zwischen der Referenzfarbe ($R_j$) und einer Referenzfarbe des ersten Textilkörpers (52).

17. Verfahren nach einem der Ansprüche 11 bis 16 wobei, zum Erkennen von Kreuzkontamination, die Fehlerfarbe (V) mit einer Differenz zwischen der Referenzfarbe ($R_j$) mehrerer verschiedener zweiter langgestreckter Textilkörper, die in der Vorrichtung verarbeitet werden, und einer Referenzfarbe des ersten Textilkörpers verglichen wird.

18. Verfahren nach einem der Ansprüche 11 bis 17, wobei die Referenzfarbe ($R_j$) eine Durchschnittsfarbe oder eine Meidanfarbe des zweiten länglichen Textilkörpers oder der zweiten länglichen Textilkörper (52) ist.

19. Verfahren nach einem der Ansprüche 11 bis 18, wobei die ersten und zweiten langgestreckten Textilkörper (52) in Garnreinigern (28) der Vorrichtung verarbeitet werden.

20. Verfahren nach einem der Ansprüche 11 bis 19, umfassend den Schritt des Speicherns der Referenzfarbe(n) ($R_j$) des zweiten länglichen Textilkörpers oder der zweiten länglichen Textilkörper (52) zusammen mit einem Ortsparameter, der einen physischen Ort angibt, an welchem der zweite längliche Textilkörper oder die zweiten länglichen Textilkörper (52) in der Vorrichtung verarbeitet wurde(n).

21. Verfahren nach einem der vorhergehenden Ansprüche, wobei der Farbparameter ($P_k$) mindestens zwei Farbkomponenten ($C_n$), insbesondere mindestens drei Farbkomponenten ($C_n$), eines Farbraums umfasst.

22. Verfahren nach Anspruch 21, wobei die Komponen-

ten ($C_n$) eine optische Reflexion oder Transmission des textilen Körpers (52) in mindestens zwei verschiedenen Spektralbereichen ($W_n$), insbesondere in mindestens drei verschiedenen Spektralbereichen ($W_n$), beschreiben, und wobei die Farbkomponenten bei drei verschiedenen Wellenlängen maximale Empfindlichkeiten ($M_n$) aufweisen.

23. Verfahren nach Anspruch 22, bei dem

die verschiedenen Spektralbereiche ($W_n$) eine gegenseitige Überlappung von weniger als 0.25 aufweisen, und/oder
mindestens ein Spektralbereich ($W_n$), insbesondere zwei davon, eine Breite von weniger als 50 nm hat oder haben, und/oder
mindestens ein Empfindlichkeitsmaximum ($M_n$) unter 550 nm und mindestens ein Empfindlichkeitsmaximum ($M_n$) über 600 nm liegt, und insbesondere mindestens ein Empfindlichkeitsmaximum ($M_n$) unter 500 nm liegt, mindestens ein Empfindlichkeitsmaximum ($M_n$) zwischen 500 nm und 600 nm liegt und mindestens ein Empfindlichkeitsmaximum ($M_n$) über 600 nm liegt.

24. Verfahren nach einem der vorhergehenden Ansprüche, wobei die Garnherstellungsvorrichtung eine Mehrzahl von Sensorköpfen (17, 18, 24, 30; 44) umfasst.

25. Verfahren nach einem der Ansprüche 21 bis 23 und nach Anspruch 24, wobei mindestens mehrere der Sensorköpfe (12, 18, 24, 30; 44) die gleichen Farbkomponenten ($C_n$) messen.

26. Verfahren nach Anspruch 25, bei dem Sensorköpfe (12, 18, 24, 30; 44), die zur Messung an verschiedenen Arten von Garnkörpern verwendet werden, die gleichen Farbkomponenten ($C_n$) messen.

27. Das Verfahren nach einem der vorhergehenden Ansprüche umfasst den Schritt der dauerhaften Speicherung der Zeitreihe von Werten.

28. Vorrichtung zur Herstellung von Garn mit

mindestens einen ersten Sensorkopf (12, 18, 24, 30; 44) und
eine Steuereinheit (31), die zur Durchführung des Verfahrens nach einem der vorhergehenden Ansprüche angepasst und ausgestaltet ist.

**Revendications**

1. Un procédé pour commander ou évaluer un processus de fabrication de fil dans un appareil de fabrication de fil ayant au moins une première tête de capteur (12, 18, 24, 30; 44), le procédé comprenant les étapes de

faire passer un corps textile allongé (52) par la première tête de capteur (12, 18, 24, 30; 44),
mesurer à une première pluralité de temps, au moyen de la première tête de capteur (12, 18, 24, 30; 44), un paramètre de couleur (Pk) du corps textile (52),
former une série temporelle de valeurs comprenant des valeurs du paramètre de couleur (Pk) attribuées à différents moments,
utiliser les valeurs du paramètre de couleur (Pk) pour commander ou évaluer le processus,
**caractérisé par le fait que** les valeurs (VPk) qui dépendent du paramètre de couleur (Pk) sont regroupées en grappes de couleurs.

2. Le procédé selon la revendication 1 comprenant en outre les étapes de

déterminer, à partir d'une variation temporelle du paramètre de couleur, au moins un paramètre de variation et
utiliser ce paramètre de variation pour commander ou évaluer le processus.

3. Le procédé selon l'une des revendications précédentes, comprenant en outre les étapes de

déterminer, à une deuxième pluralité de temps, en plus du paramètre de couleur (Pk) du corps textile (52) dans ladite série temporelle, au moins un paramètre de fonctionnement ($O_m$) de l'appareil et
traiter des valeurs dudit paramètre de fonctionnement ($O_m$) dans la série temporelle de valeurs,
et en particulier dans lequel les valeurs du paramètre de fonctionnement ($O_m$) sont stockées de manière permanente en tant que partie de la série temporelle de valeurs.

4. Le procédé selon la revendication 3, dans lequel la première pluralité de temps et la deuxième pluralité de temps s'étendent sur au moins un intervalle de temps commun.

5. Le procédé selon l'une quelconque des revendications 3 ou 4, dans lequel le paramètre de fonctionnement ($O_m$) comprend au moins un réglage de machine de l'appareil.

6. Le procédé selon la revendication 5, dans lequel le(s) réglage(s) de la machine comprend/comprennent au moins l'un des éléments suivants

un état marche-arrêt d'une section de traitement de l'appareil,
un fournisseur de matières premières,
une couleur de fil filé sur une machine voisine, et
un réglage de la machine pendant l'étirage, et
l'humidité ambiante.

7. Le procédé selon l'une quelconque des revendications 3 à 6, dans lequel l'appareil comprend plusieurs sections de traitement, et dans lequel le procédé comprend une détermination, pour plusieurs des sections de traitement, des paramètres de fonctionnement individuels ($O_m$).

8. Le procédé selon la revendication 7 dans lequel le corps textile (52) est un fil d'une couleur donnée et dans lequel les valeurs ($VO_m$) dépendant des paramètres de fonctionnement ($O_m$) des sections de traitement qui ne traitent pas le fil de la couleur donnée (52) sont corrélées avec la valeur (VPk) dépendant du paramètre de couleur (Pk) du fil.

9. Le procédé selon l'une quelconque des revendications précédentes comprenant l'étape de corrélation croisée d'une valeur ($VO_m$) dépendant du paramètre de fonctionnement ($O_m$) et d'une valeur (VPk) dépendant du paramètre de couleur (Pk).

10. Le procédé selon l'une quelconque des revendications précédentes comprenant les étapes de

   grouper, en particulier regrouper, les défauts de fil en fonction du paramètre de couleur (Pk) et/ou d'un paramètre de fonctionnement, en particulier d'un paramètre indiquant la capacité électrique et/ou le diamètre du fil, en différents groupes dans un nettoyeur de fil, et
   appliquer des règles de traitement différentes, en particulier des critères d'élimination des fils différents, pour les différents groupes.

11. Le procédé selon l'une quelconque des revendications précédentes, comprenant les étapes de

   détecter, à l'aide des valeurs du paramètre de couleur, au moins une section potentiellement défectueuse dans un premier corps textile allongé (52),
   pour détecter une contamination croisée, comparer au moins une couleur de défaut (V) dérivée du paramètre de couleur (Pk) dans la section potentiellement défectueuse à au moins une couleur de référence ($R_j$) d'un deuxième corps textile allongé (52) traité dans ledit appareil.

12. Le procédé selon la revendication 11, dans lequel le deuxième corps textile allongé (52) est traité, dans ledit appareil, en même temps que le premier corps textile (52).

13. Le procédé selon la revendication 11, dans lequel le deuxième corps textile allongé (52) a été traité, dans ledit appareil, avant le traitement du premier corps textile (52), et en particulier dans lequel

   un temps écoulé entre le traitement du deuxième et du premier corps textile (52) n'est pas supérieur à un mois, en particulier pas supérieur à une semaine, en particulier pas supérieur à un jour et/ou
   un temps écoulé entre le traitement du deuxième et du premier corps textile (52) est d'au moins une heure.

14. Le procédé selon l'une quelconque des revendications 11 à 13, dans lequel le deuxième corps textile allongé (52) a une couleur de référence ($R_j$) différente de celle du premier corps textile allongé (52).

15. Le procédé selon l'une quelconque des revendications 11 à 14 dans lequel la couleur de référence ($R_j$) est mesurée par l'appareil au moyen de la première tête de capteur (12, 18, 24, 30; 44) ou au moyen d'une deuxième tête de capteur (12, 18, 24, 30; 44) de l'appareil.

16. Le procédé selon l'une quelconque des revendications 11 à 15, dans lequel, pour comparer la couleur du défaut (V) à l'au moins une couleur de référence ($R_j$), le procédé comprend les étapes de

   déterminer une déviation (T) entre la couleur du défaut (V) et une couleur souhaitée (DC) du premier corps textile allongé (52) et
   comparer la déviation (T) à une différence entre la couleur de référence ($R_j$) et une couleur de référence du premier corps textile (52).

17. Le procédé selon l'une quelconque des revendications 11 à 16, dans lequel la couleur du défaut (V) est comparée à une différence entre la couleur de référence ($R_j$) de plusieurs deuxièmes corps textiles allongés différents traités dans ledit appareil et une couleur de référence du premier corps textile.

18. Le procédé selon l'une quelconque des revendications 11 à 17, dans lequel la couleur de référence ($R_j$) est une couleur moyenne ou une couleur médiane du ou des deuxièmes corps textiles allongés (52).

19. Le procédé selon l'une quelconque des revendications 11 à 18 dans lequel le premier et le deuxième corps textile allongé (52) sont traités dans des nettoyeurs de fils (28) de l'appareil.

20. Le procédé selon l'une quelconque des revendications 11 à 19 comprenant l'étape consistant à stocker la ou les couleurs de référence ($R_j$) du ou des deuxièmes corps textiles allongés (52) ainsi qu'un paramètre d'emplacement indiquant un emplacement physique où le ou les seconds corps textiles allongés (52) ont été traités dans l'appareil.

21. Le procédé selon l'une quelconque des revendications précédentes, dans lequel le paramètre de couleur ($P_k$) comprend au moins deux composantes de couleur ($C_n$), en particulier au moins trois composantes de couleur ($C_n$) d'un espace colorimétrique.

22. Le procédé selon la revendication 21, dans lequel les composantes ($C_n$) décrivent une réflexion ou une transmission optique du corps textile (52) dans au moins deux plages spectrales ($W_n$) différentes, en particulier dans au moins trois plages spectrales ($W_n$) différentes, et dans lequel les composantes de couleur ont des sensibilités maximales ($M_n$) à trois longueurs d'onde différentes.

23. Le procédé selon la revendication 22, dans lequel

   les différentes plages spectrales ($W_n$) ont un chevauchement mutuel inférieur à 0,25, et/ou au moins une plage spectrale ($W_n$), en particulier deux d'entre elles, a ou ont une largeur inférieure à 50 nm, et/ou au moins une sensibilité maximale ($M_n$) est inférieure à 550 nm et au moins une sensibilité maximale ($M_n$) est supérieure à 600 nm, et en particulier au moins une sensibilité maximale ($M_n$) est inférieure à 500 nm, au moins une sensibilité maximale ($M_n$) est comprise entre 500 nm et 600 nm, et au moins une sensibilité maximale ($M_n$) est supérieure à 600 nm.

24. Le procédé selon l'une quelconque des revendications précédentes, dans lequel l'appareil de fabrication de fils comprend plusieurs têtes de capteur (17, 18, 24, 30; 44).

25. Le procédé selon l'une quelconque des revendications 21 à 23 et de la revendication 24, dans lequel au moins plusieurs des têtes de capteur (12, 18, 24, 30; 44) mesurent les mêmes composantes de couleur ($C_n$).

26. Le procédé selon la revendication 25 dans lequel les têtes de capteur (12, 18, 24, 30; 44) utilisées pour mesurer différents types de corps de fil mesurent les mêmes composantes de couleur ($C_n$).

27. Le procédé selon l'une quelconque des revendications précédentes comprenant l'étape de stockage permanent de la série temporelle de valeurs.

28. Un appareil de fabrication de fils comprenant

   au moins une première tête de capteur (12, 18, 24, 30; 44) et
   une unité de commande (31) adaptée et structurée pour mettre en œuvre le procédé selon l'une quelconque des revendications précédentes.

Fig. 1

**Fig. 2**

**Fig. 3**

**Fig. 4**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3305700 A1
- EP 1123995 A1
- EP 3748343 A1